# EUROPEAN PATENT APPLICATION

(11) **EP 3 100 614 A1**
(43) Date of publication of application: **07.12.2016**
(21) Application number: 15170363.4
(22) Date of filing: 02.06.2015
(51) Int. Cl.: A23G 4/12, A61K 8/99, A61Q 11/00, A23L 33/135

(54) **COMPOSITIONS COMPRISING ANTI-CARIOGENIC BACTERIA AND FERMENTABLE SACCHARIDES**

(71) Applicant: Universiteit van Amsterdam, 1012 WX Amsterdam (NL)
(72) Inventor: Deng, Dong Mei, 1081 LA Amsterdam (NL); Crielaard, Willem, 7314 PC Apeldoorn (NL)
(74) Representative: De Vries & Metman

(57) **Abstract**

The present invention relates to compositions comprising anti-cariogenic bacteria for use in the oral cavity, which are able to minimize dental caries risk induced by sugar consumption and maintain good oral health. The compositions according to the invention comprise hydrogen peroxide-producing oral bacteria and fermentable saccharides and are able to inhibit cariogenic, acid-producing bacteria even at pH values of 5.5 or less. The probiotic compositions according to the invention are suitable for oral application and are preferably used in the oral cavity.

## Description

The present invention relates to compositions comprising anti-cariogenic bacteria for use in the oral cavity, which are able to minimize dental caries risk induced by sugar consumption and maintain good oral health.

The human oral cavity harbours a dynamic microbial community, which consists of more than 700 bacterial species [J.A. Aas et al. J Clin Microbiol 2005, 43: 5721-5732]. In a healthy situation, this community maintains a healthy microbial homeostasis, through a dynamic balance of synergistic and antagonistic microbial interactions. Disturbance of this homeostasis can lead to shifts in microbial composition and eventually cause diseases e.g. dental caries [P. Marsh, Advances in dental Research 1994, 8: 263-271]. Dental caries is still a major oral health problem in most industrialized countries, affecting 60-90% of schoolchildren and the vast majority of adults (World Health Organization). Dental caries (tooth decay) is the chemical dissolution of the dental hard tissues by the acid produced by the bacteria on the tooth surfaces after fermentation of dietary carbohydrates. When bacterially produced acids are sufficient to reduce plaque pH below the 'critical pH' (pH 5.5), the enamel will start to dissolve. Dental plaque is a multi-microbial community. Under prolonged low-pH environment, the outgrowth of acidogenic and aciduric species such as mutans streptococci and lactobacilli shifts the microbial composition and enhances the dissolution of the dental hard tissue, eventually causing caries [P.D. Marsh,BMC Oral health 2006, 6: S14; B. Nyvad et al. Caries Res 2013, 47: 89-102]. A similar situation applies to animals, in particular mammals.

Sugar consumption is one of the important risk factors for caries. But sugar is also one of the most popular food ingredients, since humans are naturally drawn to the taste of sweetness. To minimize the risk of caries while satisfying the need of human being for sweet tastes, a wide range of non-fermentable, or poorly fermentable sugar substitutes, such as sugar alcohols, e.g. sorbitol, mannitol and xylitol, have been proposed as a solution to an increasing caries problem. Many food products and drinks claim to be sugar-free by adding various sugar substitutes. However, concurrent with the fast development of sugar-substitutes, there are also concerns about the health, economic and environmental impact of these substitutes. In terms of health impact, sugar alcohols are not absorbed in the small intestine and overconsumption can lead to bloating, diarrhea and flatulence. In terms of economic impact, the natural sugar substitutes, such as e.g. Stevia, are generally more expensive than sugar, due to the limitations of the natural sources. In terms of environmental impact, the synthetic artificial sweeteners such as e.g. aspartame, cyclamate, or sucralose, though they are as cheap as sucrose, have a carbon footprint which is an estimated 4-times higher than the carbon footprint of sucrose. The synthetic artificial sweeteners are also recognized as a class of environmental contaminants due to their extreme persistence and ubiquitous occurrence in various aquatic ecosystems (Sang et al., Water Res. 2014, 52:260-274). Hence there is a need to find alternative solutions which satisfy the need of consumers for sweet taste and minimize the risk of caries.

The present invention provides a solution for this need. The present invention provides for a composition comprising hydrogen peroxide-producing oral bacteria and fermentable saccharides. Surprisingly, it was found that the addition of hydrogen peroxide-producing (HPP) oral bacteria to a composition comprising fermentable saccharides minimized the risk of caries induced by fermentation of said fermentable saccharides. The fermentable sugars cause an initial drop in pH due to the acid produced by the cariogenic bacteria on the tooth surface (dental plaque) after fermentation of the saccharide. As pH values initially dropped below the critical, cariogenic pH of 5.5, it was surprisingly found that the low pH induced an increase in the production of hydrogen peroxide by the hydrogen peroxide-producing oral bacteria, thereby inhibiting the cariogenic bacteria and the production of acid. The increased hydrogen peroxide production of these hydrogen peroxide-producing bacteria caused an overall reduction in the production of acid, and consequently shortened the low-pH period after saccharide consumption. The reduction in the production of acid is an indicator of the inhibitory effect of the hydrogen peroxide-producing bacteria. The combined administration of the hydrogen peroxide-producing oral bacteria and the fermentable saccharides will result in a shortening of the low-pH period after consumption of said saccharides, thus minimizing the risk of caries, even under cariogenic conditions. This special feature distinguishes the hydrogen peroxide-producing oral bacteria from other oral probiotic bacteria and makes them extremely suitable to lower the probability of developing dental caries and to maintain a healthy microflora in the oral cavity even in the presence of fermentable saccharides. The present invention thus provides for the combined use of hydrogen peroxide-producing oral bacteria and fermentable saccharides to minimize the risk of developing dental caries and to maintain a healthy microflora in the oral cavity even in the presence of fermentable saccharides.

The use of probiotic bacteria is a well-known concept in the prevention and treatment of dental caries. Common probiotic bacteria such as *Lactobacillus* and *Bifodobacterium* are lactic acid producing and acid-tolerant bacterial species, which in themselves are potential cariogenic bacteria and therefore not suitable for the prevention and treatment of caries. It has therefore been suggested to use probiotic compositions comprising non-cariogenic tooth-colonizing bacteria to inhibit the growth of the cariogenic mutans streptococci and lactobacilli, thus shifting the microbial composition towards a healthy oral microbial community. High numbers of non-mutans streptococci, such as *Streptococcus sanguinis,* and *Streptococcus gordornii* in the dental biofilms (plaque) were often associated with low numbers of cariogenic bacteria *Streptococcus mutans* and this association was mostly observed in healthy subjects, while the inverse was typically found in subjects with dental caries [M.R. Becker et al. J Clin Microbiol. 2002, 40: 1001-1009; R.J. Berkowitz, Journal Canadian Dental Association 2003, 69: 304-307]. Although substantial evidence has indicated that *S. sanguinis* and *S*. *gordonii* were capable of inhibiting the growth of *S. mutans,* these results were mostly achieved at a neutral pH environment. When a multi-species community was incubated under prolonged low pH condition, *S. mutans* would be favored while *S. sanguinis* and *S. gordonii* would be suppressed [D. Bradshaw et al., Caries research 2002,36: 81-86 ;D. Bradshaw et al., Caries research 1998, 32: 456-462]. Streptococcus oligofermentans has been frequently isolated from caries-free subjects or healthy non-caries tooth surfaces [Tong et al. Int J Syst Evol microbial. 2003, 53(Pt 4): 1101-1104; Zhang et al. Caries Res. 2010, 44(4): 345-348]. Although the inhibition of *S. oligofermentans* on the growth of *S. mutans* in dual-species biofilms has been reported in previous studies [J. Zang et al., Caries research 2010, 44: 345-348; L. liu et al., Appl Envir Microbiol 2012, 78:2120-2127; H. Tong et al., J Bacteriol 2008, 190: 4716-4721] these results were mostly achieved at a neutral pH environment. Moreover, a recent study found that a low pH environment (pH<6.0) affected the ability of *S. oligofermentans* to inhibit the growth of *S. mutans* [Y. Liu et al., Eur J Oral Sci 2014, 122: 57-61]. Thus, these non-mutans streptococci were found to inhibit cariogenic mutans streptococci in the dental biofilm at neutral pH environment, but not under cariogenic conditions. Hence there is still a need for probiotic compositions which are able to inhibit cariogenic bacteria at cariogenic conditions, preferably at pH values of 5.5 or less. The present invention solves this problem by the addition of fermentable saccharides to a probiotic composition comprising the non-cariogenic, hydrogen peroxide-producing streptococci. It was very surprising that the addition of fermentable saccharides enabled these bacteria to inhibit the cariogenic bacteria at cariogenic conditions. Without being bound by theory, it is believed that the initial drop in pH caused by the acid produced by the cariogenic bacteria on the tooth surface after fermentation of the saccharides triggered an increase in the production of hydrogen peroxide. The observed increase of hydrogen peroxide production was very surprising and unexpected. The high production of hydrogen peroxide not only inhibited the cariogenic bacteria but also caused a rise in pH due to inhibition of the acid production, thereby shortening the period of low-pH. The increase in production of hydrogen peroxide thus enabled the hydrogen peroxide-producing non-cariogenic oral bacteria to survive under cariogenic conditions.

Thus, in one aspect, the present invention provides for a composition comprising hydrogen peroxide-producing oral bacteria and fermentable saccharides. A composition according to the present invention comprising hydrogen peroxide-producing bacteria and fermentable saccharides is able to inhibit cariogenic, acid-producing bacteria even at pH values of 5.5 or less. The probiotic compositions according to the invention are suitable for oral application and are preferably used in the oral cavity. For the purpose of the invention, probiotic bacteria according to the invention refer to beneficial oral bacteria which after administration to the host (usually humans or other mammals including pets and livestock) help to maintain or improve the oral health of the host. The present invention is not directed to intestinally derived probiotic bacteria which are delivered orally to obtain a beneficial effect in the gut or stomach.

The present invention advantageously allows the application of fermentable saccharides instead of the various poorly fermentable or non-fermentable sugar substitutes in a food product comprising the hydrogen peroxide-producing bacteria according to the invention without imposing the risk of caries to the consumer. In a preferred aspect, the composition according to the present invention is a food product. For the purpose of the invention, food products include animal feed, preferably pet animal and livestock feed. The present invention thus provides for a food product comprising hydrogen peroxide-producing oral bacteria and fermentable saccharides. The food products according to the invention will have a reduced risk of dental caries induced by consumption of said fermentable saccharides. The food products according to the present invention will satisfy the need for sweet taste without the economical or environmental risks associated with the various sugar substitutes: they are either cheaper (compared to a Xylitol-containing product) or are environmental friendlier (compared to food products containing artificial sweeteners).

Suitable hydrogen peroxide-producing bacteria for use in the compositions and products according to the invention are non-cariogenic, oral bacteria which are able to attach to the tooth surface in the oral cavity, produce low amounts of acid following fermentation of dietary saccharides and produce hydrogen peroxide. Particularly suitable hydrogen peroxide-producing bacteria according to the invention are acid-tolerant hydrogen peroxide-producing bacteria. For the purpose of the invention, acid-tolerant bacteria are bacteria which are able to survive at pH 5.5 for at least 12 hrs, preferably at least 14 hrs, more preferably at least 16 hrs. Preferably the hydrogen peroxide producing bacteria according to the invention are acid-tolerant and capable to convert the lactic acid produced by the cariogenic bacteria into hydrogen peroxide. More preferably, the hydrogen peroxide-producing bacteria are *Streptococcus* bacteria, such as e.g. *Streptococcus oligofermentans* and *Streptococcus cristatus.* Highly preferred bacteria for use in the compositions and products of the present invention are *S. oligofermentans* bacteria due to a significant higher hydrogen peroxide production after incubation at low pH compared to hydrogen peroxide production at neutral pH. The high production of hydrogen peroxide not only inhibited the cariogenic bacteria and therefore the acid production, but also caused a rise in pH due to the conversion of lactic acid into hydrogen peroxide. This combination of features makes S. oligofermentans extremely suitable to lower the probability of developing dental caries and to maintain a healthy microflora in the oral cavity even in the presence of fermentable saccharides. For the purpose of the present invention, oral cavity is understood to refer to the oral cavity of animals as well.

Fermentable saccharides according to the invention are monosaccharides, disaccharides and mixtures thereof. For the purpose of the invention, saccharides are understood to be aldoses and ketoses, preferably D-aldoses and D-ketoses and do not include sugar alcohols such as sorbitol, mannitol, xylitol and the like. Preferably the monosaccharides is a pentose or hexose, more preferably fructose, ribulose, ribose, arabinose, xylose, lyxose, glucose, galactose, mannose, allose, altrose, talose and the like. Highly preferred are glucose, fructose and galactose or mixtures thereof. Preferably the disaccharide is sucrose (saccharose, also known as table sugar, cane sugar or beet sugar), lactose, maltose or mixtures thereof. More preferably the disaccharide is sucrose.

Suitable food products according to the invention can be low moisture food products and high moisture food products. The latter group includes dairy products such as e.g. milk, yoghurts, cheese and the like, non-dairy products such as soy milk, almond milk and the like, fermented and non-fermented beverages such as e.g. mineral water, enriched drinks, juices and the like. Low moisture food products includes confectionery products, biscuits, chewing gum and animal feed pellets. In particular chewing gum is highly preferred as a food product according to the invention comprising hydrogen peroxide-producing oral bacteria and fermentable saccharides. The daily use of a chewing gum according to the invention will assist the colonization of the hydrogen peroxide-producing bacteria in the dental plaque. The fermentable saccharides present in the chewing gum of the present invention will cause initial low-pH environment in the dental plaque, and help the hydrogen peroxide-producing bacteria to inhibit the cariogenic bacteria, thereby minimizing the risk of caries induced by the dietary saccharides. Preferably the chewing gum according to the invention comprises hydrogen peroxide-producing Streptococcus bacteria, more preferably *S. oligofermentans* and/or *S. cristatus.* More preferably, the chewing gum according to the invention comprises *S. oligofermentans* and a fermentable saccharide, preferably a saccharide selected from the group of glucose, fructose, sucrose or mixtures thereof.

Chewing gum comprising probiotic bacteria have been described. Several probiotic bacteria, such as *Lactobaccilus* (BioGaia) and *Streptococcus salivarius* (CulturedCare™ probiotic gum) have been added into chewing gums. Salivary mutans streptococci were significantly reduced when the healthy young adults took the chewing gum containing Lactobacili reuteri daily for 3 weeks (Calgar et al. Clin Oral Investig. 2007, 11(4):425-429. Epub 2007 Jun 2016). However, these commercial probiotic bacteria are in themselves strong acid producers and therefore cariogenic and not suitable to prevent dental caries. In contrast, the hydrogen peroxide-producing bacteria according to the invention, in particular the hydrogen peroxide-producing Streptococcus bacteria, more in particular *S. oligofermentans* and *S. cristatus,* produce low amounts of acid and inhibit the high acid-producing cariogenic oral bacteria.

The compositions according to the present invention are also suitable for use in dental health care to prevent, treat or reduce dental caries. In a further aspect, the present invention provides for a pharmaceutical composition for use in the oral cavity, comprising hydrogen peroxide-producing bacteria and fermentable saccharides, and a suitable pharmaceutical carrier. The present invention allows the incorporation of hydrogen peroxide-producing bacteria and fermentable saccharides in a dental health care product to prevent, treat or reduce dental caries and to maintain a healthy oral microflora. Suitable dental health care products according to the invention are toothpastes, mouthwashes, tablets and powders.

The compositions and products according to the present invention can be prepared using conventional production techniques known in the art. To obtain the favorable effect, the bacteria according to the invention need to be viable in the composition. The compositions and products of the invention generally have a viable bacterial count of at least 10⁶ colony forming units (CFU), preferably at least 10⁷ CFU, more preferably at least 10⁸ CFU per gram of composition.

The hydrogen peroxide producing bacteria can be added as a freeze dried dry powder to the compositions and products of the invention at various stages of production using standard production technologies for the specific products. Process conditions during manufacture of a composition or product according to the invention should be controlled in order to avoid conditions which would render the probiotic bacteria ineffective. Particular care should be taken to avoid high temperatures after addition of the bacteria (such as sterilization or pasteurization). The industry, and in particular the food industry has a broad experience with alternative conservation technologies such as e.g. microfiltration, to avoid protein inactivation. In addition, the compositions and product may comprise additives, stabilizers, fillers and other bulk material which are conventional for these compositions and products.

In yet a further aspect, the present invention provides for a method to prevent or reduce dental caries wherein a composition according to the invention is administered to the oral cavity of a mammal, preferably a human being, pet animal or livestock. Preferably the administration is at least once a day and carried out over a longer period of time. The hydrogen peroxide-producing oral bacteria and fermentable sugar can be administered simultaneously, or sequentially. For simultaneous administration the bacteria and saccharides according to the invention the bacteria and fermentable saccharides preferably are administered as one composition. For sequential administration the bacteria and saccharides according to the invention can be administered separately one after the other.

Thus, the invention furthermore provides for a kit of parts suitable for administration of the hydrogen peroxide-producing oral bacteria and the fermentable saccharides, comprising a composition comprising the hydrogen peroxide-producing oral bacteria and a composition comprising the fermentable saccharides.

The present invention is further illustrated by the following figures and examples without being limited thereto or thereby.

### LEGENDA

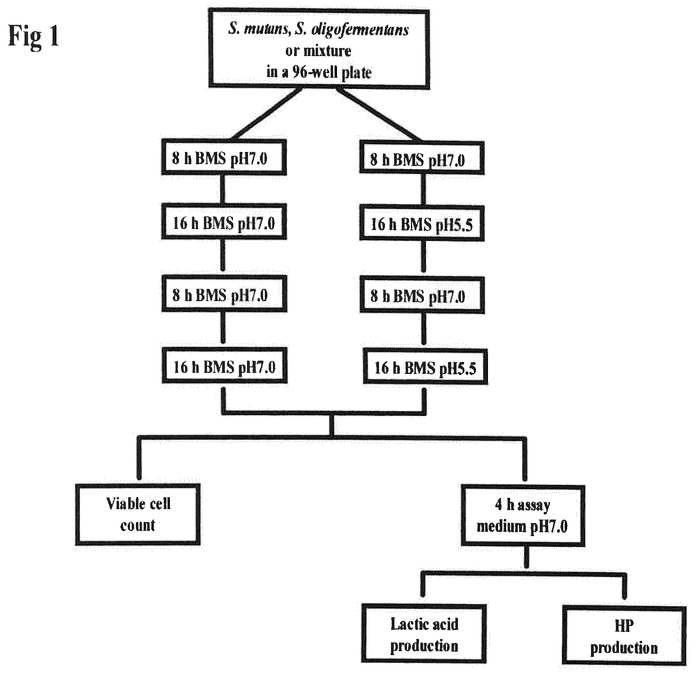

Fig. 1: The scheme of the biofilm experiment.

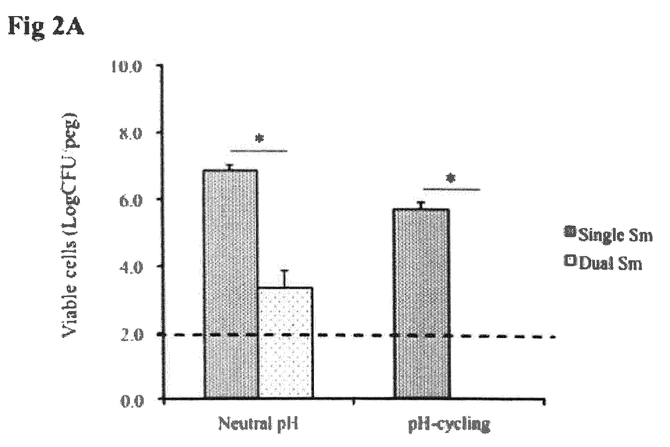

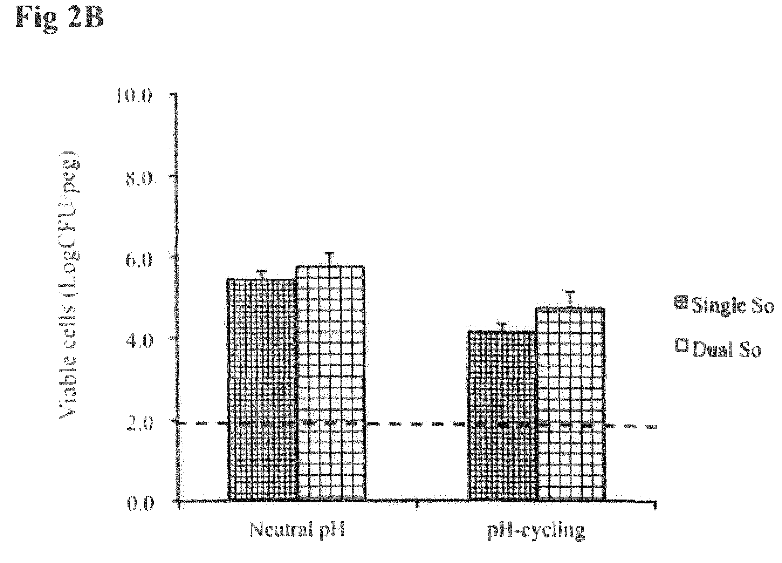

Fig. 2: Viability of single-species and dual-species 48 h biofilms. Biofilms were grown under constantly neutral pH or pH-cycling (8 h pH 7.0 and 16 h pH 5.5) conditions. The viability of the biofilms was evaluated by CFU counts. **A.** Viable cell counts of *S. mutans* biofilms. Single Sm: *S. mutans* single-species biofilms; Dual Sm: *S. mutans* counts in dual-species biofilms. **B.** Viable cell counts of *S. oligofermentans* biofilms. Single So: *S. oligofermentans* single-species biofilms; Dual So: *S. oligofermentans* counts in dual-species biofilms. The dash line indicates the detection limit. Four replicates were used for viable cell counts in each experiment. The experiment was repeated 3 times. * indicates the significant difference between Single Sm and Dual Sm, p *<* 0.05.

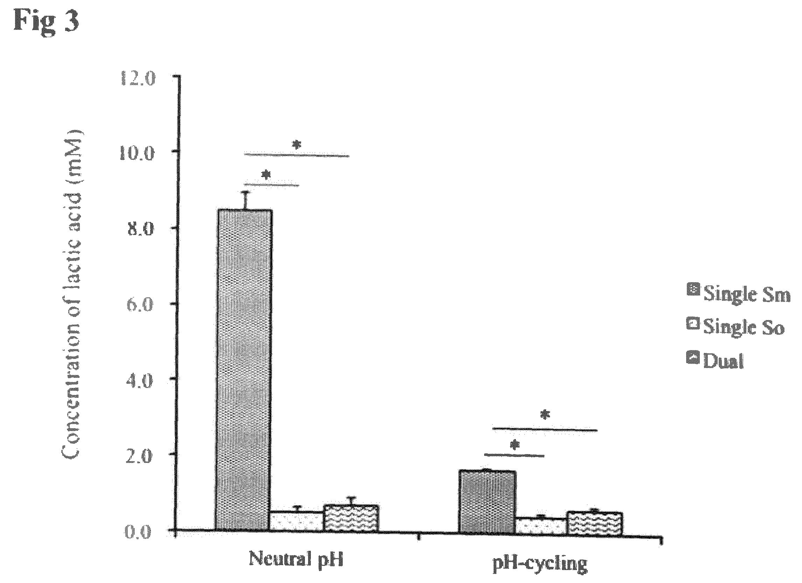

Fig. 3: Lactic acid concentration of single-species and dual-species 48 h biofilms. Biofilms were grown under constantly neutral pH or pH-cycling conditions. Before quantification of lactic acid concentration, the biofilms were incubated in the assay medium (pH 7.0), supplemented with 1% glucose for 4 h. Single Sm: *S. mutans* single-species biofilms; Single So: *S. oligofermentans* single-species biofilms; Dual: dual-species biofilms. Four replicates were used for lactic acid quantification in each experiment. The experiment was repeated 3 times. * indicates the significant difference between Single Sm and the other two groups (Single So and Dual), p < 0.05.

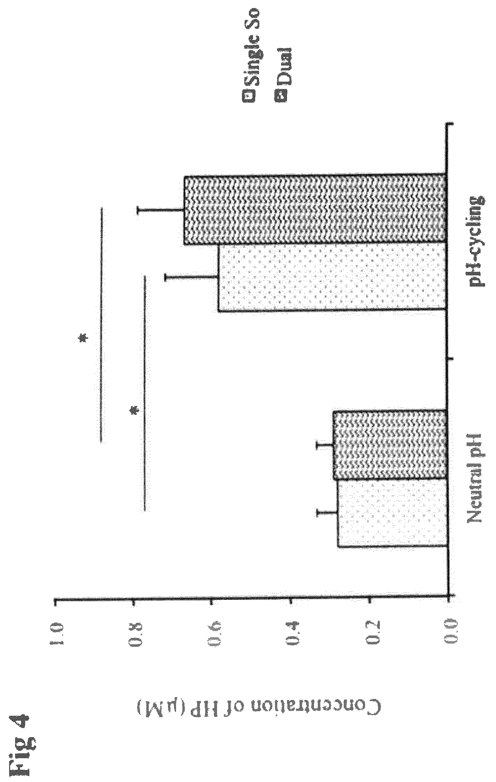

Fig. 4: Hydrogen peroxide (HP) production of single-species and dual-species 48 h biofilms. Biofilms were grown under constantly neutral pH or pH-cycling conditions. The HP production was quantified after the biofilms were incubated in the assay medium (pH 7.0), supplemented with 1% glucose for 4 h. Single So: *S. oligofermentans* single-species biofilms; Dual: dual-species biofilms. Four replicates were used for HP production in each experiment. The experiment was repeated 3 times. * indicates the significant difference between constantly neutral pH and pH-cycling conditions, p < 0.05.

### EXAMPLES

The aims of this study are to establish a pH-controllable active-attachment biofilm model and to explore the competition between *S. mutans* and *S. oligofermentans* in biofilms under two different pH conditions, constantly neutral pH and pH-cycling. The pH-cycling included a period of 8h at neutral pH and a period of 16h at pH 5.5, with the intention to mimic cariogenic conditions that dental biofilms often encounter.

### Materials and methods

### Bacterial strains and growth conditions

The strains used in this study were *Streptococcus mutans* UA159 and *Streptococcus oligofermentans* LMG22279 [H.Tong et al., International Journal of Systematic and Evulutionary Microbiology 2003, 53: 1101-1104]. Both bacterial strains were grown anaerobically (90% N₂, 5% CO₂, 5% H₂) at 37°C. Biofilms were grown in a modified semi-defined biofilm medium (BM), which contains 10 mM (NH₄)₂SO₄, 35 mM NaCl, 2 mM MgSO₄·7H₂O and was supplemented with filter-sterilised vitamins (0.04 mM nicotinic acid, 0.1 mM pyridoxine HCl, 0.01 mM pantothenic acid, 1 µM riboflavin, 0.3 µM thiamine HCl, and 0.05 µM D-biotin), amino acids (4 mM L-glutamic acid, 1 mM L-arginine HCl, 1.3 mM L-cysteine HCl, and 0.1 mM L-tryptophan), and 0.3% (wt/vol) yeast extract [D. Bradshaw et al., Caries research 2002, 36: 81-86].

To prepare BM of pH 7.0, 76 mM K₂HPO₄ and 15 mM KH₂PO₄ were added to the medium. To prepare BM of pH 5.5, 30 mM MES buffer was added to the medium. For pre-cultures, BMG was prepared by adding 0.4% of glucose to BM and for biofilm growth, BMS was prepared by adding 0.2% of sucrose to BM. This sucrose concentration was chosen because it could promote biofilm formation without causing pH changes.

### Biofilm growth

Biofilms were grown in an active attachment model [X. Li et al., J Basic Microbiol 2013, 54: 97-103]. This model consists of a standard 96-well microtiter plate and a lid with an identical number of polystyrene pegs that fit into wells (NuncTM, Roskilde, Denmark). This model was chosen to examine exclusively the actively adhered biofilms, instead of bacterial sedimentation in the 96-well microtiter plate, and to retain the 96-well high-throughput advantage for testing multiple variables. Single or dual-species biofilms were grown for 48 h before further analysis. In detail, to grow single-species biofilms, overnight (16 h) *S. mutans* and *S*. *oligofermentans* cultures in BMG were diluted to a final OD₆₀₀ₙₘ of 0.04 in fresh BMS (pH 7.0) and 200 µl of each cell suspension was dispensed into a 96-well plate. To grow dual-species biofilms, the overnight cultures of each strain were diluted to a final OD₆₀ₙₘ of 0.08 in BMS (pH 7.0) and mixed at 1:1 ratio before dispensing 200 µl into the 96-well plate. This mixture contains 4.6 x 10⁶ (± 2.7 x 10⁶) CFU/ml of *S. mutans* and 1.7 x 10⁷ (± 7.4 x10⁶) CFU/ml of *S. oligofermentans.* The plate was then covered with the lid containing pegs and incubated for 8 h. Thereafter, the pegs were rinsed with sterile distilled water to remove non-adherent bacterial cells. After rinsing, half of the pegs were inserted in BMS of pH 7.0, while the other half was inserted in BMS of pH 5.5, and both were further incubated for 16 h. Subsequently all pegs were rinsed with sterile distilled water and inserted again in BMS of pH 7.0. After another 8 h incubation, the pegs were inserted in either BMS of pH 7.0 or BMS of pH 5.5 for another 16 h. The 48 h biofilms formed on the pegs were collected for viable cell counts and were examined for their capability to produce lactic acid and HP. The schema of biofilm growth is illustrated in Fig. 1.

In each experiment, a total of 6 groups (single-/dual-species groups, grown under neutral pH or pH-cycling condition) were tested. Each group generally contained 8 biofilms replicates. Four replicates were used for viable cell counts and four were used for the measurement of lactic acid and HP production. The experiment was repeated 3 times.

### Viable cell counts

Each individual peg with biofilms was carefully cut off with a sterile scalpel without disturbing the biofilms and placed in 1ml CPW (5 g yeast extract, 1 g peptone, 8.5 g NaCl, 0.5 g L-cysteine hydrochloride per liter, adjusted to pH 7.3). Biofilms were dispersed by sonication on ice 60 times for 1 s at an amplitude of 40 W (Vibra cell™, Sonics & Materials Inc., USA). Undiluted samples or serially diluted samples (100 µl) were plated onto Brain Heart Infusion (BHI) agar plates. The plates were incubated for 3 d. The colonies were counted and recorded as Colony Forming Unit (CFU). Since the morphologies of *S. mutans* and *S. oligofermentans* were distinct on BHI agar plates, the CFUs of *S. mutans* and *S. oligofermentans* in the dual-species biofilm samples were recorded separately based on the colony morphology. The images of the colonies are provided in S1 and S2 Figs. The detection limit of this viable cell count method is 100 CFU per sample.

### HP and lactic acid quantification

The capability of HP and lactic acid production of 48 h biofilms was examined by inserting the pegs with biofilms into a 96-well plate, filled with 200 µl per well buffered assay medium (pH 7.0) at 37°C for 4 h. The assay medium contained most of the components of BM except yeast extract, since no further biofilm growth was desired during the incubation. Glucose (1%) was added to the assay medium in order to trigger lactic acid production. After incubation, 50 µl assay medium was immediately used for HP measurement. The rest of the medium was stored at -20°C for lactic acid quantification.

HP was quantified by an enzymatic assay with modifications [M Seki et al., J of bacteriology 2004, 186: 2046-2051]. In detail, 50 µl of culture medium was added to 45 µl of solution containing 2.5 mM 4-aminoantipyrine (4-amino-2, 3-dimethyl-1-phenyl-3-pyrazolin-5-one; Sigma) and 0.17 M phenol in a 96-well plate. This reaction mixture was incubated for 5 min at room temperature; thereafter horseradish peroxidase (Sigma-Aldrich, St. Louis, MO, USA) was added at a concentration of 640 mU ml⁻¹ in 0.2 M potassium phosphate buffer (pH 7.2). After 4 min, the absorbance was recorded at 510 nm in a spectrophotometer (Perkin Elmer, Norwalk, CT, USA). HP concentration of each sample was calculated from a standard curve generated with known concentrations of HP (Sigma-Aldrich, St. Louis, MO, USA).

Lactic acid was measured with an enzymatic-spectrophotometric method [I. Gutmann et al., Methods of enzymatic analysis. 1974 New York: Academic Press, pp 1464]. The principle of the method is based on the enzymatic conversion of L-lactate to pyruvate with concomitant conversion of NAD to NADH, the increase in absorbance at 340 nm being proportional to NADH formation.

### HP production of S. oligofermentans suspension cells at pH 7.0 or pH 5.5

To understand how environmental pH influenced the ability of HP production of *S. oligofermentans,* we chose to carry out the experiment on suspension cells since these cells were more homogenous and better controlled than the biofilm cells. Overnight *S. oligofermentans* culture grown in BMG (pH 7.0) was centrifuged and resuspended in either BM pH7.0 or BM pH5.5, without any addition of sucrose. The cell density was adjusted to an OD₆₀₀ₙₘ of 0.9. The resuspensions were incubated anaerobically for either 4 or 16 h. The ability of these suspension cells to produce HP was examined in the same way as the biofilms were tested. In detail, the resuspensions were centrifuged, incubated in the buffered assay medium for 4 h. The supernatants were used for HP quantification (the procedure is described above). The *S. oligofermentans* cells before and after 4 or 16 h of incubation were also subjected to viable cell counts. This experiment was repeated 3 times. Duplicate samples per group were included in each experiment.

### Statistical analysis

The data were analysed with the Statistical Package for Social Science (SPSS, Version 17.0, Chicago, IL, USA). One-way ANOVA was used to evaluate the differences in CFU, HP and lactic acid concentration between single- and dual-species biofilms. Specifically, independent-samples t test was used to examine the influence of pH (neutral pH vs. pH-cycling) on the variables. The CFU counts were log transformed before the statistical tests. *p*<0.05 was considered significant.

### Results

### Viable cell counts of single- and dual-species biofilms

Both *S. mutans* and *S. oligofermentans* were able to form biofilms alone and together in the presence of 0.2% sucrose. At every refreshment point the pH of the spent medium was monitored. After 16 h incubation, the pH of phosphate buffered biofilm medium (pH7.0) had remained constant, but the pH of MES buffered medium (pH 5.5) dropped 0.5 unit.

Fig. 2 displays the CFU counts of single- and dual-species biofilms. Compared to constantly neutral pH condition, pH 7.0-5.5 cycling condition generally lead to one log reduction in the total cell counts of 48 h biofilms. The presence of *S. oligofermentans* in dual-species biofilms significantly suppressed the growth of *S. mutans.* The cell counts of *S. mutans* dropped from 10⁶ CFU/peg in single-species biofilms to 10³ CFU/peg in dual-species biofilms at constantly neutral pH, and dropped from 10⁵ CFU/peg to under the detection limit (10² CFU/peg) at pH-cycling conditions. However, the presence of *S. mutans* in dual-species biofilms did not seem to affect *S*. *oligofermentans* cell counts at tested pH conditions. The number of *S. oligofermentans* viable cells in dual-species biofilms was similar to that in single-species biofilms at either tested pH.

### Lactic acid concentration of single- and dual-species biofilms

The growth pH conditions affected only the lactic acid concentration of single-species *S. mutans* biofilms (Fig. 3). The pH-cycling condition, compared to neutral pH condition, significantly reduced lactic acid production of single-species *S. mutans* biofilms (p<0.05). The lactic acid concentration in dual-species biofilms was significantly lower than that in single-species *S. mutans* biofilms. The reduction was around 12-fold under constantly neutral pH and 2.7-fold under pH-cycling condition. Similar level of acid concentration was observed in dual-species and single-species *S. oligofermentans* biofilms.

### HP production of single- and dual-species biofilms

No HP production was measured for single-species *S. mutans* biofilms. We also examined the suspension cells of *S. mutans* UA159 grown in either BHI or BMS (pH 7.0) and found no HP production. This experiment was performed twice with duplicate samples (data not shown). Therefore HP in the biofilms was produced by *S. oligofermentans* alone (Fig. 4). There were no differences in HP production between dual-species and single-species *S. oligofermentans* biofilms. The HP production in the biofilms grown at the pH-cycling condition was 2-fold higher than those in the biofilms grown at constantly neutral pH.

### HP production by S. oligofermentans suspension cells

To further explore if the increased HP production of *S. oligofermentans* under the pH-cycling condition was stress-related, we measured HP production of *S. oligofermentans* suspension cells after they were incubated in a buffer of pH 7.0 or pH 5.5 for 4 or 16 h. Table 1 shows that the CFU counts significantly dropped after incubating the cell suspension in a buffer of pH 5.5 for 16 h. No changes in CFU counts were observed under other incubation conditions. The *S. oligofermentans* suspension cells incubated in a buffer of pH 5.5 for 16 h demonstrated the highest potential of producing HP. As a result, the amount of HP produced per viable cell under this condition was about 15-fold higher than those incubated under other conditions.

**Table 1. Viable cell counts and HP production of S. oligofermentans suspension cells after 4 or 16 h low pH challenge***

| Period | pH | Before incubation (logCFU) | After incubation (logCFU) | HP production (nM/viable cell) |
|---|---|---|---|---|
| 4 h | 7.0 | 8.0±0.1 | 7.9±0.1 | 4.3±0.3 |
| | 5.5 | 8.0±0.1 | 7.9±0.1 | 4.3±0.8 |
| 16 h | 7.0 | 7.7±0.1 | 7.6±0.1 | 8.0±0.5 |
| | 5.5 | 7.6±0.1 | 6.5±0.1** | 116.5±15.4** |

| | | | | |
|---|---|---|---|---|
| * Data are presented as average ± sd. ** Statistical significance when compared to the corresponding values under neutral pH condition after 16 h incubation (p < 0.01). | | | | |

### Discussion

In this study, the competition between the cariogenic *S*. *mutans* and the commensal *S. oligofermentans* in dual-species biofilms was examined under either a constantly neutral pH or a pH-cycling condition (with a 16 h low pH period) in an active attachment biofilm model. Our data demonstrated that *S. oligofermentans* strongly inhibited the growth of *S. mutans* in dual-species biofilms and reduced lactic acid concentration at not only neutral pH condition, but also the pH-cycling condition.

In our study, the oscillation of pH between 7.0 and 5.5 allowed the growth of both bacterial species. This design mimics the clinical condition better and makes it possible to study the competition between both species. Overall, our finding implied that *S. oligofermentans* might be a compelling probiotic candidate to compete against cariogenic bacterial species at sites prone to caries.

*S. mutans* is known to be acid tolerant. The data presented here suggested that *S. oligofermentans* could inhibit *S. mutans* even after co-incubation at low pH for 16 h. This trait was indicated by the relatively high CFU counts of single-species *S. oligofermentans* biofilms under pH-cycling condition. It seems that *S. oligofermentans* is as acid resistant as *S. mutans,* since a similar level of CFU reduction was observed for both bacterial species when comparing pH-cycling condition with neutral pH condition.

Another intriguing finding of this study was the enhanced HP production of *S. oligofermentans* biofilms after growth under pH-cycling conditions. This was unexpected since the number of viable *S. oligofermentans* cells grown under pH-cycling conditions was much lower than of those grown under neutral pH conditions. There are a few possible explanations. The first one is that the presence of *S. mutans* in dual-species biofilms might trigger the HP production under pH-cycling conditions. But it could not explain why the HP production in single-species *S. oligofermentans* biofilms was as high as dual-species biofilms. The second explanation is that the high HP production was caused by the high lactate concentration, since *S. oligofermentans* is able to convert lactate into HP. This assumption could explain the low lactate concentration observed in *S. oligofermentans* biofilms. But it could not explain why lactate concentration in *S. oligofermentans* biofilms was similar under both pH conditions, while HP production was different. The third explanation is related to the plating method used in this study. Since this method cannot detect viable but not culturable bacterial cells, the viable but not culturable *S. oligofermentans* cells in the biofilms might be the reason for the high HP production. We have carried out the metabolism (resazurin) assay to examine the metabolic activity of the tested biofilms, besides plating. This assay is able to detect viable bacterial cells without the requirement of further culturing. The results obtained from the assay were consistent with the results obtained from the plating method (data not shown). The fourth explanation is that the prolonged low pH incubation triggered the stress response of *S. oligofermentans* cells and resulted in enhanced HP production after the bacterial cells were incubated at neutral pH again. To explore this assumption, we carried out an additional experiment on *S. oligofermentans* suspension cells. Our data indicated 16 h, but not 4 h, mild acid pre-challenge could indeed increase the HP production per *S. oligofermentans* cell, even though the overall HP increase was not as pronounced as in biofilms.

In this study, we successfully established a high-throughput pH-cycling biofilm model. In order to establish a controllable pH-cycling condition, different buffers (phosphate or MES buffer) were added to the growth media. As a result, the pH of the biofilms could be maintained as designed. Since dental biofilms in the oral cavity constantly experience pH fluctuations, the established model can mimic this fluctuation condition and help to understand its influence on the bacterial interaction in dental biofilms.

In summary, the data presented here indicated that the non-mutans streptococci *S. oligofermentans* is low acidogenic, a hydrogen peroxide producer, aciduric and able to produce a substantial amount of HP after substantial acid challenge in the biofilms. These traits make *S. oligofermentans* the most competent candidate to compete against cariogenic bacteria *S*. *mutans in vivo.*

## Claims

1. A probiotic composition comprising hydrogen peroxide-producing oral bacteria and a fermentable saccharide.

2. A composition according to claim 1 for the inhibition of cariogenic bacteria at pH 5.5 or less.

3. A composition according to claim 1 or 2, wherein said composition is a food product.

4. A food product according to claim 3, wherein said food product is a gum based confectionary product, preferably a chewing gum.

5. A composition according to claim 1 or 2, wherein said composition is a pharmaceutical composition for use in the oral cavity.

6. A pharmaceutical composition according to claim 5 for the inhibition of cariogenic bacteria at pH 5.5 or less.

7. A composition according to claim 1 or 2, wherein said composition is a dental health care composition for use in the oral cavity.

8. A dental health care composition according to claim 7 for inhibiting cariogenic bacteria at pH 5.5 or less.

9. A food product or a composition according to any of the preceding claims wherein the hydrogen peroxide-producing oral bacteria are *Streptococcus* bacteria.

10. A food product or a composition according to claim 9, wherein the hydrogen peroxide-producing oral bacteria are selected from the group of *S.cristatus* and *S*. *oligofermentans.*

11. A food product or a composition according to any of the preceding claims wherein the hydrogen peroxide-producing oral bacteria is *S. oligofermentans.*

12. A food product or a composition according to any of the preceding claims wherein the saccharide is a mono- or disaccharide or a mixture thereof.

13. A food product or a composition according to any of the preceding claims wherein the saccharide is glucose, fructose, or sucrose or mixtures thereof.

14. Use of a composition for the oral cavity comprising hydrogen peroxide-producing oral bacteria and fermentable saccharides for the prevention or reduction of dental caries.

15. Use of a composition for the oral cavity comprising hydrogen peroxide-producing oral bacteria for the inhibition of cariogenic bacteria at pH 5.5 or less.

16. Use of a dental care product comprising hydrogen peroxide-producing oral bacteria and fermentable saccharides for the inhibition of cariogenic bacteria at pH 5.5 or less.

17. A kit comprising a composition comprising hydrogen peroxide-producing oral bacteria and a composition comprising fermentable saccharides.

18. A kit according to claim 17 for the prevention or reduction of dental caries

19. A kit according to claim 17 for the inhibition of cariogenic bacteria at pH 5.5 or less.
